## Europäisches Patentamt
## European Patent Office
## Office européen des brevets

(11) Veröffentlichungsnummer: **0 558 997 B1**

(12) **EUROPÄISCHE PATENTSCHRIFT**

(45) Veröffentlichungstag der Patentschrift: **22.11.95**

(51) Int. Cl.6: **C07C 17/00**, C07C 25/00

(21) Anmeldenummer: **93102462.4**

(22) Anmeldetag: **17.02.93**

(54) **Verfahren zur Verminderung des Halogengehaltes von polyhalogenierten Aromaten.**

(30) Priorität: **02.03.92 DE 4206530**

(43) Veröffentlichungstag der Anmeldung:
**08.09.93 Patentblatt 93/36**

(45) Bekanntmachung des Hinweises auf die
Patenterteilung:
**22.11.95 Patentblatt 95/47**

(84) Benannte Vertragsstaaten:
**BE DE FR GB IT NL**

(56) Entgegenhaltungen:

CHEMISTRY LETTERS. Nr. 10, Oktober 1987,
TOKYO JP Seiten 2051 - 2052 KIYONORI SHI-
NODA 'Transchlorination of o-Dichlorobenzene and Benzene into Chlorobenzene'

CHEMICAL ABSTRACTS, vol. 112, no. 23, 4.
Juni 1990, Columbus, Ohio, US; abstract no.
216311f, KIYONORI SHINODA ET AL. 'Trans-
chlorination of polychlorobenzenes and
benzene into chlorobenzene' Seite 584
;Spalte 2 ;

PATENT ABSTRACTS OF JAPAN vol. 14, no.
106 (C-694)(4049) 27. Februar 1990 & JP-A-13
11 032

(73) Patentinhaber: **BAYER AG**

**D-51368 Leverkusen (DE)**

(72) Erfinder: **Schrage, Heinrich, Dr.**
**Dörperhofstrasse 31**
**W-4150 Krefeld 1 (DE)**
Erfinder: **Fiege, Helmut, Dr.**
**Walter-Flex-Strasse 23**
**W-5090 Leverkusen 1 (DE)**

**Beschreibung**

Die vorliegende Erfindung betrifft ein Verfahren zur Verminderung des Halogengehaltes von polyhalogenierten Aromaten, bei dem polyhalogenierte Aromaten mit monohalogenierten und/oder nicht halogenierten Aromaten zur Umsetzung gebracht werden mit dem Ziel, Halogenatome vom polyhalogenierten Aromaten auf den monohalogenierten und/oder nicht halogenierten Aromaten zu übertragen und gleichzeitig Wasserstoffatome vom monohalogenierten und/oder nicht halogenierten Aromaten auf den polyhalogenierten Aromaten zu übertragen.

Aus der japanischen Patentanmeldung mit der Offenlegungsnummer 01-311.032 ist die Umsetzung von polychlorierten Aromaten (z.B. 1,2-Dichlorbenzol und 1,2,4-Trichlorbenzol) mit Benzol bekannt. Als Katalysator wird Aktivkohle eingesetzt, die mit Palladiumchlorid und einem Seltenerdmetallchlorid beladen ist. Bei dieser Umsetzung entstehen Gemische aus den beiden Ausgangsprodukten (z.B. 1,2-Dichlorbenzol und Benzol) mit deren Reaktionsprodukt (z.B. Chlorbenzol) und mit Isomerisierungsprodukten des polychlorierten Ausgangsmaterials (z.B. 1,3- und 1,4-Dichlorbenzol).

Aus Chemistry Letters 1987, 2051 ist die Umsetzung von 1,2-Dichlorbenzol mit Benzol in Gegenwart von mit Chloriden des Iridiums, Rhodiums oder Osmiums beladener Aktivkohle bekannt. Diese Katalysatoren zeigen allerdings eine wesentlich schlechtere chlorübertragende Wirkung als die mit Palladiumchlorid/Seltenerdmetallchlorid beladenen Aktivkohlen.

Aus der EP-OS 256 479 ist die Umsetzung von Polyjodbenzol (z.B. 1,4-Dijodbenzol) mit Benzol bekannt. Als Katalysatoren werden Zeolithe des Typs X und Y eingesetzt, die Thallium oder Seltenerdmetalle enthalten können. Das Reaktionsprodukt ist ein Gemisch aus dem im Überschuß eingesetzten Benzol mit Jodbenzol.

Es wurde nun ein Verfahren zur Verminderung des Halogengehaltes von polyhalogenierten Aromaten durch Umsetzung von polyhalogenierten Aromaten mit monohalogenierten und/oder nicht halogenierten Aromaten in der Gasphase gefunden, bei dem Halogenatome vom polyhalogenierten Aromaten auf den monohalogenierten und/oder nicht halogenierten Aromaten und gleichzeitig Wasserstoffatome vom monohalogenierten und/oder nicht halogenierten Aromaten auf den polyhalogenierten Aromaten übertragen werden, das dadurch gekennzeichnet ist, daß man die Reaktion in Gegenwart eines Katalysators, der mindestens ein Element aus der Gruppe Ruthenium, Rhodium, Palladium, Osmium, Iridium und Platin in elementarer Form oder in Form von Verbindungen enthält und in Gegenwart von mindestens 0,1 Gew.-% Halogenwasserstoff, bezogen auf die Menge des eingesetzten Aromatengemischs, durchführt.

In das erfindungsgemäße Verfahren können als polyhalogenierte Aromaten beispielsweise solche eingesetzt werden, die als Substituenten nur Halogenatome oder Halogenatome und weitere Substituenten enthalten. Weitere Substituenten können beispielsweise 1 bis 2 $C_1$- bis $C_6$-Alkylreste sein.

Bei den polyhalogenierten Aromaten kann es sich beispielsweise um solche auf der Basis von Benzol, Naphthalin oder Biphenyl handeln, insbesondere um polyhalogenierte Benzole, polyhalogenierte Mono- und Dialkylbenzole, polyhalogenierte Naphthaline und polyhalogenierte Biphenyle. Die polyhalogenierten Aromaten enthalten pro Molekül mindestens zwei und maximal soviel Halogenatome, wie am jeweiligen aromatischen System maximal als Substituenten vorhanden sein können. Sonst keine Substituenten enthaltende polyhalogenierte Benzole können also 2 bis 6 Halogenatome enthalten, polyhalogenierte Monoalkylbenzole 2 bis 5 Halogenatome, polyhalogenierte Dialkylbenzole 2 bis 4 Halogenatome, sonst keine Substituenten enthaltende polyhalogenierte Naphthaline 2 bis 8 Halogenatome und sonst keine Substituenten enthaltende polyhalogenierte Biphenyle 2 bis 10 Halogenatome.

Als Halogen können die polyhalogenierten Aromaten z.B. Chlor, Brom und/oder Jod enthalten. Bevorzugt enthalten sie Chlor und/oder Brom, ganz besonders bevorzugt Chlor.

Die polyhalogenierten Aromaten können in einem Molekül untereinander gleiche, aber auch verschiedene Halogenatome enthalten. Bevorzugt sind polyhalogenierte Aromaten, die nur eine Sorte Halogenatome enthalten, beispielsweise nur Chloratome.

Beispiele für erfindungsgemäß einsetzbare polyhalogenierte Aromaten sind: 1,2-, 1,3- und 1,4-Dichlorbenzol, 1,2,3-, 1,2,4- und 1,3,5-Trichlorbenzol, 1,2,3,4- und 1,2,4,5-Tetrachlorbenzol, Pentachlorbenzol, Hexachlorbenzol, die verschiedenen isomeren Di-, Tri- und Tetrachlortoluole, die verschiedenen isomeren Dibrombenzole, die verschiedenen isomeren Tribrombenzole, die verschiedenen isomeren Tetrabrombenzole, die verschiedenen isomeren Dijodbenzole, die verschiedenen isomeren Di-, Tri- und Tetrachlornaphthaline, die verschiedenen isomeren Di-, Tri- und Tetrachlorbiphenyle und die verschiedenen isomeren Di-, Tri- und Tetrabrombiphenyle.

In das erfindungsgemäße Verfahren können in sich einheitliche polyhalogenierte Aromaten eingesetzt werden. Bevorzugt werden jedoch Gemische verschiedener polyhalogenierter Aromaten eingesetzt, beispielsweise Gemische aus Tri- und Tetrachlorbenzolen mit kleinen Anteilen (z.B. jeweils unter 10 Gew.-%)

EP 0 558 997 B1

an Penta- und Hexachlorbenzol, oder Gemische chlorierter Naphthaline mit einem mittleren Chlorierungsgrad von beispielsweise 4 bis 6 oder Gemische chlorierter Biphenyle mit einem mittleren Chlorierungsgrad von beispielsweise 3 bis 6.

Es können auch Gemische von polyhalogenierten Aromaten unterschiedlichen Typs eingesetzt werden, beispielsweise polyhalogenierte Benzole im Gemisch mit polyhalogenierten Naphthalinen oder polyhalogenierte Benzole im Gemisch mit polyhalogenierten Biphenylen. Dies ist jedoch eine weniger bevorzugte Variante.

Sofern gemischte polyhalogenierte Aromaten eingesetzt werden ist der Anteil der einzelnen Komponenten solcher Gemische im allgemeinen ohne Belang.

In das erfindungsgemäße Verfahren können als monohalogenierte und/oder nicht halogenierte Aromaten beispielsweise solche Aromaten eingesetzt werden, die keine Substituenten, einen Halogensubstituenten, einen Halogensubstituenten und 1 bis 2 $C_1$- bis $C_6$-Alkylgruppen oder nur 1 bis 2 $C_1$- bis $C_6$-Alkylgruppen enthalten. Bei den monohalogenierten und nicht halogenierten Aromaten kann es sich beispielsweise um Benzole, Alkylbenzole, Naphthaline oder Biphenyle handeln. Die monohalogenierten Aromaten können als Halogen Fluor, Chlor, Brom oder Jod enthalten. Bevorzugt wird unsubstituiertes Benzol eingesetzt.

Das Einsatzverhältnis von polyhalogenierten Aromaten zu monohalogenierten und/oder nicht halogenierten Aromaten kann im Rahmen der vorliegenden Erfindung prinzipiell beliebig gewählt werdend Aus wirtschaftlichen Erwägungen ist es jedoch bevorzugt, den monohalogenierten und/oder nicht halogenierten Aromaten in einem molaren Überschuß einzusetzen, beispielsweise in einer Menge von 1 bis 10 Mol pro Mol Äquivalent austauschbarer Halogenatome am polyhalogenierten Aromaten.

Als Halogenwasserstoff ist bei der vorliegenden Erfindung Chlorwasserstoff und Bromwasserstoff bevorzugt. Besonders bevorzugt ist der Halogenwasserstoff, der das gleiche Halogen enthält, das im umzusetzenden polyhalogenierten Aromaten überwiegend vorhanden ist.

Der Halogenwasserstoff wird mindestens in einer Menge von 0,1 Gew.-%, bezogen auf die Menge des eingesetzten Aromatengemisches, zugefügt. Vorzugsweise beträgt diese Menge 5 bis 200 Gew.-%, insbesondere 10 bis 100 Gew.-%. Wenn man zusätzlich zu Halogenwasserstoff ein inertes Gas einsetzt, ist es häufig ausreichend, Halogenwasserstoff in einer Menge von 5 bis 50 Gew.-%, bezogen auf eingesetztes Aromatengemisch, einzusetzen.

Erfindungsgemäß einzusetzende Katalysatoren enthalten mindestens ein Element aus der Gruppe Ruthenium, Rhodium, Palladium, Osmium, Iridium und/oder Platin in elementarer Form oder in Form von Verbindungen. Bevorzugt sind lösliche, insbesondere wasserlösliche Platinmetall-Salze, Besonders bevorzugt sind elementares Palladium und Palladiumverbindungen, beispielsweise Palladiumdichlorid.

Es ist im allgemeinen vorteilhaft die Platinmetalle oder deren Verbindungen auf einem Trägermaterial aufgebracht zum Einsatz zu bringen. Ein bevorzugtes Trägermaterial ist Aktivkohle, die gegebenenfalls gekörnt sein kann.

Für das erfindungsgemäße Verfahren besonders geeignete Katalysatoren kann man z.B. erhalten, wenn man gekörnte Aktivkohle mit der wäßrigen Lösung eines Salzes eines Metalls der Platinmetallgruppe tränkt und anschließend trocknet. Die so auf die Aktivkohle aufgebrachten Salze können gegebenenfalls modifiziert werden, beispielsweise durch eine oder mehrere Nachbehandlungen, beispielsweise mit wäßrigen Lösungen von Natriumchlorid, Ammoniak, Hydrazin und/oder Säuren.

Nach der Aufbringung und Trocknung der wäßrigen Lösung eines Salzes eines Metalls der Platinmetallgruppe kann man so erhaltene Kontakte auch modifizieren, indem man noch mit der Lösung eines oder mehrerer anderer Metallsalze tränkt und anschließend trocknet. Solche anderen Metallsalze können beispielsweise Salze anderer Platingruppen-Metalle und/oder anderer Metalle sein, z.B. Salze von Barium, Eisen und/oder Kupfer, insbesondere deren löslichen Chloride. Man kann auch nur eine Tränkung mit einer wäßrigen Lösung mehrerer Metallsalze vornehmen, in der jedoch mindestens ein Salz eines Platingruppen-Metalls enthalten ist.

Erfindungsgemäß einzusetzende Katalysatoren können z.B. 0,05 bis 50 Gramm Elemente aus der Gruppe der Platinmetalle in elementarer Form oder in Form von Verbindungen, bezogen auf 1 l des Trägermaterials enthalten. Vorzugsweise liegt diese Menge bei 0,1 bis 10 Gramm.

Bezogen auf eingesetzte polyhalogenierte Aromaten können z.B. 0,005 bis 10 Gew.-% Elemente aus der Gruppe der Platinmetalle in elementarer Form oder in Form von Verbindungen eingesetzt werden.

Wenn man Katalysatoren einsetzt, die ein Salz eines Metalls der Platinmetallgruppe und weitere Metallsalze enthalten, so kann dabei das molare Verhältnis von Platinmetallsalz zu Co-Metallsalzen beispielsweise 1:10 bis 10:1 betragen. Vorzugsweise liegt dieses Verhältnis im Bereich 2:1 bis 1:2.

Das erfindungsgemäße Verfahren ist ein Gasphasen-Verfahren. Der feste heterogene Katalysator kann dabei in einer technisch üblichen Anordnung angewendet werden, z.B. im Festbett oder im Wirbelbett.

3

Neben den gasförmigen Einsatzstoffen und deren gasförmigen Reaktionsprodukten können im Reaktionsraum gegebenenfalls noch unter den Reaktionsbedingungen inerte Gase vorhanden sein, beispielsweise Stickstoff und/oder Argon.

Das erfindungsgemäße Verfahren kann bei Normaldruck, erniedrigtem Druck oder erhöhtem Druck durchgeführt werden. Bevorzugt ist Normaldruck oder ein erhöhter Druck, beispielsweise ein Druck bis zu 30 bar.

Die Reaktionstemperaturen sind in einem weiten Bereich variierbar. Nach unten ist die Reaktionstemperatur dadurch begrenzt, daß bei dem gewählten Reaktionsdruck alle Einsatzstoffe und deren Reaktionsprodukte in gasförmiger Phase vorliegen müssen. Nach oben ist die Reaktionstemperatur dadurch begrenzt, daß bei zu hoher Temperatur unerwünschte Nebenreaktionen in einem unerwünschten Maß eintreten können, z.B. Reaktionen, bei denen aromatische Kerne gespalten werden und/oder Verharzungen auftreten, die zur Desaktivierung des Katalysators führen können. Im allgemeinen kann man das erfindungsgemäße Verfahren bei Reaktionstemperaturen zwischen 200 und 600°C, bevorzugt 300 bis 500°C durchführen.

Eine beispielhafte Ausführungsform des erfindungsgemäßen Verfahrens ist folgende:

Man stellt einen Katalysator aus einer auf Holzkohle basierenden, mit Dampf aktivierten und gut gewaschenen körnigen Aktivkohle her, der 5 g pro Liter eines Platinmetalls in Form des Chlorids enthält. Nach dem Trocknen wird dieser Katalysator mit einer weiteren Metallsalzlösung getränkt und erneut getrocknet. Der Katalysator, der nun zwei Salze, z.B. in einem Molverhältnis von 1:1 enthält, wird in ein Reaktionsrohr gegeben, bei dem das Verhältnis des Durchmessers zum Durchmesser der Katalysatorteilchen 20:1 bis 50:1 beträgt. Anschließend wird der Katalysator 2 Stunden bei der gewünschten Temperatur (z.B. 400°C) in einem Inertgasstrom aufgeheizt. Danach wird eine Mischung eines polyhalogenierten Aromaten mit einem monohalogenierten und/oder nicht halogenierten Aromaten, z.B. isomere Trichlorbenzole und Benzol, gasförmig aus einer Vorverdampfungszone über eine Vorheizzone unter Zusatz von Chlorwasserstoff (beispielsweise 50 Gew.-% bezogen auf das eingesetzte Aromatengemisch) über den auf die gewünschte Temperatur vorgeheizten Katalysator geleitet. Gegebenenfalls kann man zusätzlich zum Halogenwasserstoff ein inertes Gas, z.B. Stickstoff, einblasen. Das gasförmige Produktgemisch wird durch eine kurze Nachheizzone geführt und durch Kühlung kondensiert. Der Halogenwasserstoff kann gegebenenfalls in den Prozeß zurückgeführt werden.

Das erfindungsgemäße Verfahren ermöglicht die Übertragung von Halogenatomen eines polyhalogenierten Aromaten auf einen mono- und/oder nicht halogenierten Aromaten mit guten Ausbeuten und deutlich verbesserten Katalysatorstandzeiten.

Die erfindungsgemäß geschaffene Möglichkeit den Halogengehalt von polyhalogenierten Aromaten zu verringern ist beispielsweise von Interesse für die Entsorgung von hochchlorierten Benzolen, die als Nebenprodukte bei der Chlorierung von Benzol zu Mono- und Dichlorbenzol entstehen und für die Enthalogenierung und damit Entsorgung von polyhalogenierten Naphthalinen und Biphenylen. Gleichzeitig kann man bei der Entchlorierung von polychlorierten Benzolen durch Umsetzung mit Benzol noch Chlorbenzol und gegebenenfalls Dichlorbenzole als weiter verwertbare Produkte gewinnen.

Beispiele

Beispiel 1 (nicht erfindungsgemäß)

Herstellung von Katalysatoren

a) 1 Liter einer dampfaktivierten Kornaktivkohle (Typ: SC XII 12 x 30; Chemviron; Korngröße ca. 1 mm; Schüttgewicht ca. 520 g/l) wurde mit soviel einer Lösung, die Palladiumchlorid und Bariumchlorid enthielt, getränkt, daß der Katalysator nach dem Trocknen bei 100°C im Vakuum 7,5 g Palladium und 9,7 g Barium pro Liter als Chlorid enthielt.

b) Es wurde wie in Beispiel 1a) verfahren, jedoch wurde 1 Liter einer Formaktivkohle (Typ WS IV spez.; Degussa; Korndurchmesser über 3,2 mm; Schüttgewicht ca. 400 g/l) eingesetzt. Die getrocknete Kohle wurde anschließend zerkleinert und die Bestandteile von 0,8 bis 3,35 mm herausgesiebt.

c) 1 Liter einer dampfaktivierten Torfkohle (Typ ROX 0,8; Norit; Korndurchmesser ca. 0,8 mm; Schüttgewicht ca. 390 g/l) wurde mit soviel einer Lösung von Palladiumchlorid getränkt, daß der Katalysator nach dem Trocknen bei 100°C im Vakuum 5,0 g Palladium pro Liter als Chlorid enthielt.

d) Die in Beispiel 1b) beschriebene Aktivkohle wurde mit soviel einer Lösung von Palladiumchlorid getränkt, daß der Katalysator nach dem Trocknen 5,0 g Palladium pro Liter enthielt. Der getrocknete Katalysator wurde nun mit einer Lösung von 17,5 g $CeCl_3 \cdot 7\,H_2O$ in 170 ml Wasser getränkt und bei 100°C im Vakuum getrocknet.

Beispiele 2 bis 5

In einem senkrecht stehenden Reaktionsrohr wurden in getrennten Ansätzen jeweils 75 ml der im Beispiel 1 beschriebenen Katalysatoren eingefüllt. Man erhielt ein Katalysatorfestbett von ca. 10 cm Höhe, das von außen beheizt wurde, Zunächst wurde das Katalysatorbett 2 Stunden in einem Stickstoffstrom von 20 l/h bei 400°C ausgeheizt. Dann wurde mit einer Geschwindigkeit von 16,5 ml/h (das entspricht 15,3 g/h) ein Gemisch aus 13,5 g 1,2,4-Trichlorbenzol und 76,5 g Benzol in einen Vorverdampfer flüssig eindosiert und dort verdampft, auf 400°C vorgeheizt und durch das Katalysatorbett bei 400°C geleitet. Gleichzeitig wurde Chlorwasserstoff in einer Menge von 4,7 l/h und Stickstoff in einer Menge von 7 l/h eingeblasen. Nach 24 bzw. 48 Stunden wurde noch 1 Stunde in einem Stickstoffstrom von 7 l/h nachgeheizt. Die Abgase wurden in einer Kühlfalle bei ca. 0°C kondensiert. Nach jeweils 4 Stunden wurde die Kühlfalle gewechselt und das Kondensat mittels einer geeichten gaschromatografischen Methode analysiert. Zum Vergleich wurde der jeweilige Versuch mit dem entsprechenden Kontakt wiederholt und dabei kein Chlorwasserstoffgas, sondern nur 11,7 l/h Stickstoff eingeblasen. Die Ergebnisse sind in den nachfolgenden Tabellen zusammengestellt.

Beispiel 2a (erfindungsgemäß)

Katalysator aus Beispiel 1a, Zufuhr von HCl und $N_2$.

| ...Stunden nach Reaktionsbeginn gesammeltes Produkt | Produktionszusammensetzung (Gew.-%) | | | |
|---|---|---|---|---|
| | Benzol | Monochlor-benzol | Dichlor-benzole | 1,2,4-Trichlor-benzol |
| 0 bis 4 | 73,7 | 26,2 | 0,1 | - |
| 20 bis 24 | 77,8 | 18,1 | 4,1 | - |
| 44 bis 48 | 82,5 | 8,4 | 6,7 | 2,4 |
| | erhaltene Mengen (g) | | | |
| 0 bis 48 | 566,2 | 111,0 | 24,7 | 2,8 |

Beispiel 2b (zum Vergleich)

Katalysator aus Beispiel 1a, nur Zufuhr von $N_2$.

| ...Stunden nach Reaktionsbeginn gesammeltes Produkt | Produktionszusammensetzung (Gew.-%) | | | |
|---|---|---|---|---|
| | Benzol | Monochlor- benzol | Dichlor- benzole | 1,2,4- Trichlor- benzol |
| 0 bis 4 | 84,0 | 16,0 | - | - |
| 20 bis 24 | 87,6 | 1,0 | 1,4 | 10,0 |
| | erhaltene Mengen (g) | | | |
| 0 bis 24 | 310,0 | 18,6 | 8,7 | 14,9 |

Beispiel 3a (erfindungsgemäß)

Katalysator aus Beispiel 1b, Zufuhr von HCl und $N_2$.

| ...Stunden nach Reaktionsbeginn gesammeltes Produkt | Produktionszusammensetzung (Gew.-%) | | | |
|---|---|---|---|---|
| | Benzol | Monochlor- benzol | Dichlor- benzole | 1,2,4- Trichlor- benzol |
| 0 bis 4 | 74,5 | 25,4 | 0,1 | - |
| 20 bis 24 | 76,3 | 20,0 | 3,7 | - |
| | erhaltene Mengen (g) | | | |
| 0 bis 24 | 274,3 | 81,6 | 1,9 | - |

6

Beispiel 3b (zum Vergleich)

Katalysator aus Beispiel 1b, nur Zufuhr von N$_2$.

| ...Stunden nach Reaktionsbeginn gesammeltes Produkt | Produktionszusammensetzung (Gew.-%) | | | |
|---|---|---|---|---|
| | Benzol | Monochlor-benzol | Dichlor-benzole | 1,2,4-Trichlor-benzol |
| 0 bis 4 | 87,3 | 9,3 | 3,2 | 0,2 |
| 20 bis 24 | 86,8 | 0,2 | 0,2 | 12,8 |
| | erhaltene Mengen (g) | | | |
| 0 bis 24 | 314,4 | 6,6 | 4,5 | 20,0 |

Beispiel 4a (erfindungsgemäß)

Katalysator aus Beispiel 1c, Zufuhr von HCl und N$_2$.

| ...Stunden nach Reaktionsbeginn gesammeltes Produkt | Produktionszusammensetzung (Gew.-%) | | | |
|---|---|---|---|---|
| | Benzol | Monochlor-benzol | Dichlor-benzole | 1,2,4-Trichlor-benzol |
| 0 bis 4 | 75,7 | 24,3 | – | – |
| 20 bis 24 | 77,3 | 18,9 | 3,8 | – |
| | erhaltene Mengen (g) | | | |
| 0 bis 24 | 282,3 | 73,9 | 7,6 | – |

Beispiel 4b (zum Vergleich)

Katalysator aus Beispiel 1c, nur Zufuhr von $N_2$.

| ...Stunden nach Reaktionsbeginn gesammeltes Produkt | Produktionszusammensetzung (Gew.-%) | | | |
|---|---|---|---|---|
| | Benzol | Monochlor-benzol | Dichlor-benzole | 1,2,4-Trichlor-benzol |
| 0 bis 4 | 87,7 | 12,3 | - | - |
| 20 bis 24 | 82,6 | 2,7 | 4,3 | 10,4 |
| erhaltene Mengen (g) | | | | |
| 0 bis 24 | 277,7 | 18,5 | 12,4 | 9,4 |

Beispiel 5a (erfindungsgemäß)

Katalysator aus Beispiel 1d, Zufuhr von HCl und $N_2$.

| ...Stunden nach Reaktionsbeginn gesammeltes Produkt | Produktionszusammensetzung (Gew.-%) | | | |
|---|---|---|---|---|
| | Benzol | Monochlor-benzol | Dichlor-benzole | 1,2,4-Trichlor-benzol |
| 0 bis 4 | 79,2 | 20,3 | 0,5 | - |
| 20 bis 24 | 84,9 | 8,3 | 5,7 | 1,1 |
| erhaltene Mengen (g) | | | | |
| 0 bis 24 | 292,4 | 46,8 | 12,5 | 0,5 |

Beispiel 5b (zum Vergleich)

Katalysator aus Beispiel 1d, nur Zufuhr von $N_2$.

| ...Stunden nach Reaktionsbeginn gesammeltes Produkt | Produktionszusammensetzung (Gew.-%) | | | |
|---|---|---|---|---|
| | Benzol | Monochlor-benzol | Dichlor-benzole | 1,2,4-Trichlor-benzol |
| 0 bis 4 | 90,4 | 6,9 | 2,7 | - |
| 20 bis 24 | 88,1 | 0,2 | 0,3 | 11,4 |
| | erhaltene Mengen (g) | | | |
| 0 bis 24 | 311,7 | 6,2 | 4,8 | 22,5 |

Beispiel 6 (erfindungsgemäß)

Beispiel 3a wurde wiederholt, jedoch wurde statt des Trichlorbenzol/Benzol-Gemisches 16,5 ml pro Stunde eines Gemisches aus 23,0 g 1,4-Dichlorbenzol und 77,0 g Benzol eingesetzt.

| ...Stunden nach Reaktionsbeginn gesammeltes Produkt | Produktionszusammensetzung (Gew.-%) | | | |
|---|---|---|---|---|
| | Benzol | Monochlor-benzol | Dichlor-benzole | 1,2,4-Trichlor-benzol |
| 0 bis 4 | 70,7 | 27,8 | 1,5 | - |
| 20 bis 24 | 73,4 | 16,0 | 10,6 | - |
| | erhaltene Mengen (g) | | | |
| 0 bis 24 | 273,7 | 68,7 | 24,7 | - |

Beispiel 7 (erfindungsgemäß)

Beispiel 3a wurde wiederholt, jedoch wurde statt des Trichlorbenzol/Benzol-Gemisches 16,5 ml pro Stunde eines Gemisches aus 10,0 g 1,2,4,5-Tetrachlorbenzol und 90,0 g Benzol eingesetzt.

| ...Stunden nach | Produktionszusammensetzung (Gew.-%) | | | |
|---|---|---|---|---|
| Reaktionsbeginn | Benzol | Monochlor- | Dichlor- | 1,2,4- |
| gesammeltes | | benzol | benzole | Trichlor- |
| Produkt | | | | benzol |
| 0 bis 4 | 89,7 | 10,3 | - | - |
| 20 bis 24 | 89,1 | 5,0 | 1,2 | 4,0 |
| | erhaltene Mengen (g) | | | |
| 0 bis 24 | 317,8 | 25,3 | 3,0 | 3,1 |

Tetrachlorbenzol wurde im Kondensat nicht gefunden.

Beispiel 8 (erfindungsgemäß)

Beispiel 3a wurde wiederholt, jedoch wurde statt des Trichlorbenzol/Benzol-Gemisches 16,5 ml pro Stunde eines Gemisches aus 0,8 g Hexachlorbenzol und 99,2 g Benzol eingesetzt.

| ...Stunden nach | Produktionszusammensetzung (Gew.-%) | |
|---|---|---|
| Reaktionsbeginn | Benzol | Monochlor- |
| gesammeltes | | benzol |
| Produkt | | |
| 0 bis 4 | 98,9 | 1,1 |
| 20 bis 24 | 99,1 | 0,9 |
| | erhaltene Mengen (g) | |
| 0 bis 24 | 337,2 | 2,4 |

Di- und höhere Chlorbenzole wurden im Kondensat nicht gefunden.

Beispiel 9 (erfindungsgemäß)

Beispiel 4a wurde wiederholt, jedoch wurde statt des Trichlorbenzol/Benzol-Gemisches 16,5 ml pro Stunde eines Gemisches aus 12 g 1,2,4-Trichlorbenzol und 88 g Chlorbenzol eingesetzt.

| ...Stunden nach Reaktionsbeginn gesammeltes Produkt | Produktionszusammensetzung (Gew.-%) | | | |
|---|---|---|---|---|
| | Benzol | Monochlor-benzol | Dichlor-benzole | 1,2,4-Trichlor-benzol |
| 0 bis 4 | 9,9 | 60,8 | 28,4 | 0,9 |
| 20 bis 24 | 3,8 | 74,3 | 21,6 | 0,3 |
| | erhaltene Mengen (g) | | | |
| 0 bis 24 | 26,5 | 308,8 | 103,8 | 1,7 |

Beispiel 10 (erfindungsgemäß)

Beispiel 3a wurde wiederholt, jedoch wurde statt des Trichlorbenzol/Benzol-Gemisches 16,5 ml pro Stunde eines Gemisches aus 10,4 g 1,3,5-Tribrombenzol und 89,6 g Benzol eingesetzt.

| ...Stunden nach Reaktionsbeginn gesammeltes Produkt | Produktionszusammensetzung (Gew.-%) | | |
|---|---|---|---|
| | Benzol | Monochlor-benzol | Brom-benzol |
| 0 bis 4 | 91,5 | 5,1 | 3,4 |
| 20 bis 24 | 87,1 | 0,1 | 12,9 |
| | erhaltene Mengen (g) | | |
| 0 bis 24 | 315,0 | 9,9 | 27,0 |

**Patentansprüche**

1. Verfahren zur Verminderung des Halogengehaltes von polyhalogenierten Aromaten durch Umsetzung von polyhalogenierten Aromaten mit monohalogenierten und/oder nicht halogenierten Aromaten in der Gasphase, bei dem Halogenatome von polyhalogenierten Aromaten auf den monohalogenierten und/oder nichthalogenierten Aromaten und gleichzeitig Wasserstoffatome vom monohalogenierten und/oder nichthalogenierten Aromaten auf den polyhalogenierten Aromaten übertragen werden, dadurch gekennzeichnet, daß man die Reaktion in Gegenwart eines Katalysators, der mindestens ein Element aus der Gruppe Ruthenium, Rhodium, Palladium, Osmium, Iridium und Platin in elementarer Form oder in Form von Verbindungen enthält, und in Gegenwart von mindestens 0,1 Gew.-% Halogenwasserstoff, bezogen auf die Menge des eingesetzten Aromatengemisches, durchführt.

2. Verfahren nach Anspruch 1, dadurch gekennzeichnet, daß man als polyhalogenierte Aromaten polyhalogenierte Benzole, polyhalogenierte Mono- oder Dialkylbenzole, polyhalogenierte Naphthaline oder polyhalogenierte Diphenyle einsetzt.

11

EP 0 558 997 B1

**3.** Verfahren nach Ansprüchen 1 und 2, dadurch gekennzeichnet, daß man als polyhalogenierte Aromaten 1,2-, 1,3- oder 1,4-Dichlorbenzol, 1,2,3-, 1,2,4- oder 1,3,5-Trichlorbenzol, 1,2,3,4-oder 1,2,4,5-Tetrachlorbenzol, Pentachlorbenzol, Hexachlorbenzol, die verschiedenen isomeren Di-, Tri- oder Tetrachlortoluole, die verschiedenen isomeren Di-brombenzole, die verschiedenen isomeren Tribrombenzole, die verschiedenen isomeren Tetrabrombenzole, die verschiedenen isomeren Diiodbenzole, die verschiedenen Isomeren Di-, Tri- oder Tetrachlornaphthaline, die verschiedenen isomeren Di-, Tri- oder Tetrachlordiphenyle oder die verschiedenen Isomeren, Di-, Tri- oder Tetrabrombiphenyle einsetzt.

**4.** Verfahren nach Ansprüchen 1 bis 3, dadurch gekennzeichnet, daß man Gemische verschiedener polyhalogenierter Aromaten einsetzt.

**5.** Verfahren nach Ansprüchen 1 bis 4, dadurch gekennzeichnet, daß man als monohalogenierte und/oder nichthalogenierte Aromaten solche Aromaten einsetzt, die keine Substituenten, einen Halogensubstituenten, einen Halogensubstituenten und eins bis zwei $C_1$-$C_6$-Alkylgruppen oder nur ein bis zwei $C_1$-$C_6$-Alkylgruppen enthalten.

**6.** Verfahren nach Ansprüchen 1 bis 5, dadurch gekennzeichnet, daß man den monohalogenierten und/oder nichthalogenierten Aromaten in einer Menge von 1 bis 10 Mol pro Mol Äquivalent austauschbarer Halogenatome am polyhalogenierten Aromaten einsetzt.

**7.** Verfahren nach Ansprüchen 1 bis 6, dadurch gekennzeichnet, daß man Halogenwasserstoff in einer Menge von 5 bis 200 Gew.-%, bezogen auf die Menge des eingesetzten Aromatengemisches, zufügt.

**8.** Verfahren nach Ansprüchen 1 bis 7, dadurch gekennzeichnet, daß der Katalysator weitere Metallsalze enthält.

**Claims**

**1.** Process for decreasing the halogen content of polyhalogenated aromatics by reaction of polyhalogenated aromatics with monohalogenated and/or non-halogenated aromatics in the gas phase, in which halogen atoms are transferred from polyhalogenated aromatics to the monohalogenated and/or non-halogenated aromatic, and at the same time hydrogen atoms are transferred from the monohalogenated and/or non-halogenated aromatic to the polyhalogenated aromatic, characterised in that the reaction is carried out in the presence of a catalyst which contains at least one element from the group comprising ruthenium, rhodium, palladium, osmium, iridium and platinum in elemental form or in the form of compounds, and in the presence of at least 0.1% by weight of hydrogen halide, based on the amount of the mixture of aromatics employed.

**2.** Process according to Claim 1, characterised in that polyhalogenated benzenes, polyhalogenated mono- or dialkylbenzenes, polyhalogenated naphthalenes or polyhalogenated diphenyls are employed as the polyhalogenated aromatics.

**3.** Process according to Claims 1 and 2, characterised in that 1,2-, 1,3- or 1,4-dichlorobenzene, 1,2,3-, 1,2,4- or 1,3,5-trichlorobenzene, 1,2,3,4- or 1,2,4,5-tetrachlorobenzene, pentachlorobenzene, hexachlorobenzene, the various isomeric di-, tri- or tetrachlorotoluenes, the various isomeric dibromobenzenes, the various isomeric tribromobenzenes, the various isomeric tetrabromobenzenes, the various isomeric diiodobenzenes, the various isomeric di-, tri- or tetrachloronaphthalenes, the various isomeric di-, tri- or tetrachlorodiphenyls or the various isomeric di-, tri- or tetrabromobiphenyls are employed as the polyhalogenated aromatics.

**4.** Process according to Claims 1 to 3, characterised in that mixtures of various polyhalogenated aromatics are employed.

**5.** Process according to Claims 1 to 4, characterised in that those aromatics which contain no substituents, one halogen substituent, one halogen substituent and one to two $C_1$-$C_6$-alkyl groups or only one to two $C_1$-$C_6$-alkyl groups are employed as the monohalogenated and/or non-halogenated aromatics.

12

6.  Process according to Claims 1 to 5, characterised in that the monohalogenated and/or nonhalogenated aromatics are employed in an amount of 1 to 10 mol per molar equivalent of replaceable halogen atoms on the polyhalogenated aromatic.

7.  Process according to Claims 1 to 6, characterised in that the hydrogen halide is added in an amount of 5 to 200% by weight, based on the amount of the mixture of aromatics employed.

8.  Process according to Claims 1 to 7, characterised in that the catalyst contains other metal salts.

**Revendications**

1.  Procédé pour diminuer la teneur en halogènes de composés aromatiques polyhalogénés par réaction de ces composés avec des composés aromatiques monohalogénés et/ou non halogénés en phase gazeuse, dans lequel on transfère des atomes d'halogènes des composés aromatiques polyhalogénés aux composés aromatiques monohalogénés et/ou non halogénés et simultanément on fait passer des atomes d'hydrogène des composés aromatiques monohalogénés et/ou non halogénés aux composés aromatiques polyhalogénés, caractérisé en ce que l'on effectue la réaction en présence d'un catalyseur contenant au moins un élément du groupe du ruthénium, du rhodium, du palladium, de l'osmium, de l'iridium et du platine à l'état élémentaire ou à l'état de dérivés, et en présence d'au moins 0,1% en poids d'un halogénure d'hydrogène, par rapport à la quantité du mélange de composés aromatiques mis en oeuvre.

2.  Procédé selon la revendication 1, caractérisé en ce que l'on utilise en tant que composés aromatiques polyhalogénés des benzènes polyhalogénés, des mono- ou di-alkylbenzènes polyhalogénés, des naphtalènes polyhalogénés ou des diphényles polyhalogénés.

3.  Procédé selon les revendications 1 et 2, caractérisé en ce que l'on utilise en tant que composés aromatiques polyhalogénés le 1,2-, le 1,3 ou le 1,4-dichlorobenzène, le 1,2,3-, le 1,2,4- ou le 1,3,5-trichlorobenzène, le 1,2,3,4- ou le 1,2,4,5-tétrachlorobenzène, le pentachlorobenzène, l'hexachlorobenzène, les diversdi-, tri- ou tétrachlorotoluènes isomères, les divers dibromobenzènes isomères, les divers tribromobenzènes isomères, les divers tétrabromobenzènes isomères, les divers diodobenzènes isomères, les divers di-, tri- et tétrachloronaphtalènes isomères, les divers di-, tri- et tétrachlorodiphényles isomères ou les divers di-, tri- ou tétrabromobiphényles isomères.

4.  Procédé selon les revendications 1 à 3, caractérisé en ce que l'on met en oeuvre des mélanges de divers composés aromatiques polyhalogénés.

5.  Procédé selon les revendications 1 à 4, caractérisé en ce que l'on utilise en tant que composés aromatiques monohalogénés et/ou non halogénés de tels composés aromatiques ne portant pas de substituant, portant un substituant halogéno, portant un substituant halogéno et un à deux groupes alkyle en $C_1$-$C_6$ ou portant uniquement un à deux groupes alkyle en $C_1$-$C_6$.

6.  Procédé selon les revendications 1 à 5, caractérisé en ce que l'on met en oeuvre les composés aromatiques monohalogénés et/ou non halogénés en quantité de 1 à 10 mol/équivalent molaire d'atomes d'halogènes échangeables des composés aromatiques polyhalogénés.

7.  Procédé selon les revendications 1 à 6, caractérisé en ce que l'on ajoute un halogénure d'hydrogène en quantité de 5 à 200% du poids du mélange de composés aromatiques mis en oeuvre.

8.  Procédé selon les revendications 1 à 7, caractérisé en ce que le catalyseur contient des sels d'autres métaux.